# FASCICULE DE BREVET EUROPEEN

(11) **EP 0 904 045 B1**
(45) Date de publication et mention de la délivrance du brevet: **12.03.2003**
(21) Numéro de dépôt: 97918243.3
(22) Date de dépôt: 16.04.1997
(51) Int. Cl.: A61K 7/06, A61K 7/50

(54) **COMPOSITIONS COSMETIQUES DETERGENTES A USAGE CAPILLAIRE ET UTILISATION**
KOSMETISCHE HAARWASCHMITTEL UND IHRE VERWENDUNG
COSMETIC DETERGENT COMPOSITIONS FOR CAPILLARY USE AND UTILISATION

(30) Priorité: 06.05.1996 FR 9605643
(43) Date de publication de la demande: 31.03.1999
(73) Titulaire: L'OREAL, 75008 Paris (FR)
(72) Inventeur: DECOSTER, Sandrine, F-93800 Epinay-sur-Seine (FR); BEAUQUEY, Bernard, F-92110 Clichy (FR)
(74) Mandataire: Le Blainvaux Bellegarde, Françoise
(86) Numéro de dépôt international: FR9700686
(87) Numéro de publication internationale: WO97041827

(56) Documents cités:
- EP-A- 0 400 976
- EP-A- 0 463 780
- EP-A- 0 468 721
- EP-A- 0 530 974
- WO-A-93/08787
- WO-A-94/03152
- US-A- 4 954 335

## Description

La présente invention concerne un procédé de préparation de compositions cosmétiques à propriétés améliorées destinées simultanément au nettoyage et au conditionnement des cheveux, et comprenant, dans un support cosmétiquement acceptable, une base lavante constituée de tensioactifs à pouvoir détergent particuliers dans laquelle sont également présents à titre d'agents conditionneurs, des polymères cationiques particuliers en association avec des silicones insolubles particulières. L'invention concerne aussi l'utilisation desdites compositions dans l'application cosmétique susmentionnée.

Pour le nettoyage et/ou le lavage des cheveux, l'utilisation de compositions capillaires détergentes (ou shampooings) à base essentiellement d'agents tensioactifs classiques de type notamment anioniques, non-ioniques et/ou amphotères, mais plus particulièrement de type anioniques, est courante. Ces compositions sont appliquées sur cheveux mouillés et la mousse générée par massage ou friction avec les mains permet, après rinçage à l'eau, l'élimination des diverses salissures initialement présentes sur les cheveux.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur la fibre capillaire des dommages plus ou moins marqués liés en particulier à l'élimination progressive des lipides ou protéines contenues dans ou à la surface de cette dernière.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Les agents conditionneurs les plus couramment utilisés à ce jour dans des shampooings sont les polymères cationiques, les silicones et/ou les dérivés de silicone, qui confèrent en effet aux cheveux lavés, secs ou mouillés, une facilité de démêlage, une douceur et un lissage nettement accrus par rapport à ce qui peut être obtenu avec les compositions nettoyantes correspondantes qui en sont exemptes.

Le document EP 400 976 décrit des compositions de shampooing contenant un tensioactif, une silicone insoluble non volatile et un agent de suspension, comme un ester d'acide gras. Le tensioactif peut être de type bétaïne, comme une alkylbétaïne ou une alkylamidobétaïne. La silicone peut être indifféremment émulsionnée ou non. La composition peut comprendre, de manière optionnelle, un agent de conditionnement cationique, comme un dérivé de cellulose ou de gomme de guar.

Le document EP 463 780 concerne une composition de shampooing contenant un tensioactif, une silicone insoluble non volatile, un agent de suspension et du mica. Le tensioactif peut être de type bétaïne, comme une alkylbétaïne en C8-C18 ou une alkylamidobétaïne en C8-C18. La silicone peut être indifféremment émulsionnée ou non. La composition peut comprendre, de manière optionnelle, un agent de conditionnement cationique, comme un dérivé de cellulose ou de gomme de guar.

Sur des cheveux sensibilisés, afin d'obtenir les effets cosmétiques des silicones sur toute la longueur de la fibre capillaire, on utilise de préférence des associations de silicones et de polymères cationiques.

Toutefois, et malgré les progrès réalisés récemment dans le domaine des shampooings à base de polymères cationiques et de silicones, ces derniers ne donnent pas vraiment complètement satisfaction, de sorte qu'un fort besoin existe encore actuellement quant à pouvoir disposer de nouveaux produits présentant, au niveau de l'une ou de plusieurs des propriétés cosmétiques évoquées ci-avant, de meilleures performances.

La présente invention vise à la satisfaction d'un tel besoin.

Ainsi, à la suite d'importantes recherches menées sur la question, il a maintenant été trouvé par la Demanderesse, de façon totalement inattendue et surprenante, qu'en utilisant une base lavante particulière, à savoir une base lavante associant au moins un tensioactif de type anionique et au moins un tensioactif amphotère de type alkylbétaïne en C₁₀-C₁₄, comprenant en outre simultanément des silicones particulières convenablement sélectionnées, telles que définies ci-après, et des polymères cationiques particuliers, à savoir des gommes de guar, à titre d'agents conditionneurs, il est possible d'obtenir des compositions détergentes présentant d'excellentes propriétés cosmétiques, en particulier au niveau de la facilité de coiffage, du maintien, de la nervosité, du lissage et de la souplesse des cheveux traités, ainsi qu'un très bon pouvoir lavant intrinsèque.

Sans vouloir limiter la présente invention à une quelconque théorie, il semblerait exister entre le polymère cationique, la silicone, le tensioactif anionique, le tensioactif amphotère conformes à l'invention et les cheveux, des interactions et/ou des affinités particulières qui favorisent un dépôt régulier, important et durable de ladite silicone et/ou dudit polymère cationique à la surface desdits cheveux, ce dépôt qualitatif et quantitatif étant probablement l'une des causes de l'amélioration observée au niveau des propriétés cosmétiques finales, en particulier la facilité de démêlage des cheveux mouillés ou séchés, la facilité de coiffage, et le lissage de la racine à la pointe des cheveux traités. En tout état de cause, les propriétés cosmétiques attachées aux compositions comprenant un tensioactif anionique, un tensioactif amphotère de type alkyl bétaïne et l'association d'agents conditionneurs [gomme de guar cationique/silicone spécifique] conforme à l'invention sont nettement supérieures à celles qui peuvent être obtenues en mettant en oeuvre des compositions ne comprenant pas la présence simultanée de tous ces composés essentiels.

Toutes ces découvertes sont à la base de la présente invention.

Ainsi, selon la présente invention, il est maintenant proposé un procédé de préparation de compositions capillaires détergentes et conditionnantes comprenant, dans un milieu cosmétiquement acceptable, (A) une base lavante comprenant au moins un tensioactif anionique et au moins un tensioactif amphotère de type alkylbétaïne en C₁₀-C₁₄, et (B) un système conditionneur comprenant au moins une gomme de guar cationique et au moins une silicone insoluble de viscosité inférieure ou égale à 350 Pa.s (350 000 cSt), choisie parmi :
(i) les polydialkylsiloxanes, (ii) les polydiarylsiloxanes et (iii) les polyalkylarylsiloxanes,
ladite silicone étant introduite dans la composition sous forme non émulsionnée.

L'invention a également pour objet l'utilisation en cosmétique des compositions ci-dessus pour le nettoyage et le conditionnement des cheveux.

Mais d'autres caractéristiques, aspects et avantages de l'invention apparaîtront encore plus clairement à la lecture de la description qui va suivre ainsi que des exemples concrets, mais nullement limitatifs, destinés à l'illustrer.

Comme indiqué précédemment, les constituants essentiels rentrant dans la composition des produits capillaires de l'invention sont (A) une base lavante comprenant au moins un tensioactif détergent anionique et (ii) au moins un tensioactif amphotère de type alkylbétaïne en C₁₀-C₁₄, (B) un système conditionneur comprenant (i) la ou les gommes de guar cationiques, et (ii) la ou les silicones insolubles.

### A- BASE LAVANTE :

Les compositions conformes à l'invention comprennent nécessairement une base lavante, généralement aqueuse.

Le ou les tensioactifs formant la base lavante comprennent un ou plusieurs tensioactifs anioniques et un ou plusieurs tensioactifs amphotères alkylbétaïne en C₁₀-C₁₄.

La quantité minimale de base lavante est celle juste suffisante pour conférer à la composition finale un pouvoir moussant et/ou détergent satisfaisant, et des quantités trop importantes de base lavante n'apportent pas vraiment d'avantages supplémentaires.

Ainsi, selon l'invention, la base lavante peut représenter de 4 % à 50 % en poids, de préférence de 10 % à 35 % en poids, et encore plus préférentiellement de 12 % à 25 % en poids, du poids total de la composition finale.

Selon une caractéristique préférée des compositions capillaires selon la présente invention, la base lavante ne contient pas d'autres tensioactifs que des tensioactifs anioniques et des tensioactifs amphotères de type alkylbétaïne en C₁₀-C₁₄.

### (i) Tensioactif(s) anionique(s) :

Leur nature ne revêt pas, dans le cadre de la présente invention, de caractère véritablement critique.

Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique,- les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides éthers carboxyliques polyoxyalkylénés et leurs sels, en particulier ceux comportant de 2 à 50 groupements oxyde d'éthylène, et leurs mélanges. Les tensioactifs anioniques du type acides ou sels d'éthers carboxyliques polyoxyalkylénés sont en particulier ceux qui répondent à la formule (1) suivante : 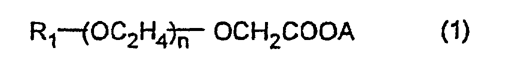 dans laquelle :
R₁ désigne un groupement alkyle ou alkylaryle, et n est un nombre entier ou décimal (valeur moyenne) pouvant varier de 2 à 24 et de préférence de 3 à 10, le radical alkyle ayant entre 6 et 20 atomes de carbone environ, et aryle désignant de préférence phényle,
A désigne H, ammonium, Na, K, Li, Mg ou un reste monoéthanolamine ou triéthanolamine. On peut également utiliser des mélanges de composés de formule (1) en particulier des mélanges dans lesquels les groupements R₁ sont différents.

Parmi tous ces tensioactifs anioniques, on préfère utiliser plus particulièrement les sels d'alkylsulfates et d'alkyléthersulfates, ainsi que leurs mélanges.

### (ii) Tensioactif(s) amphotère(s) :

Selon l'invention, les agents tensioactifs amphotères doivent être choisis parmi les alkyl (C₁₀-C₁₄) bétaïnes de formule : 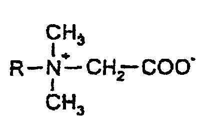 dans laquelle R désigne un radical alkyle, linéaire ou ramifié, en C₁₀-C₁₄, et de préférence en C₁₂-C₁₄.

En particulier, on préfère utiliser la cocoylbétaïne vendue par la société HENKEL sous la dénomination DEHYTON AB 30.

### B- SYSTEME CONDITIONNEUR

### (i)- Gomme(s) de guar cationique(s) :

Les compositions conformes à l'invention comprennent en outre nécessairement une gomme de guar cationique.

De manière générale, au sens de la présente invention, on entend par "gomme de guar cationique" toute gomme de guar contenant des groupements cationiques et/ou des groupements ionisables en groupements cationiques.

Les groupements cationiques préférés sont choisis parmi ceux comportant des groupements amine primaires, secondaires, tertiaires et/ou quaternaires.

Les gommes de guar cationiques utilisées ont généralement une masse moléculaire moyenne en poids comprise entre 500 et 5.10⁶ environ, et de préférence comprise entre 10³ et 3.10⁶ environ.

Les gommes de guar cationiques utilisables selon la présente invention sont par exemple des gommes de guar comportant des groupements cationiques trialkylammonium. De préférence, 2 à 30 % en nombre des fonctions hydroxyle de ces gommes de guar porte des groupements cationiques trialkylammonium.

Parmi ces groupements trialkylammonium, on peut tout particulièrement citer les groupements triméthylammonium et triéthylammonium.

Encore plus préférentiellement, ces groupements représentent de 5 à 20 % en poids du poids total de la gomme de guar modifiée.

Selon l'invention, on utilise de préférence une gomme de guar modifiée par des groupements hydroxypropyl triméthylammonium.

Ces gommes de guar modifiées par des groupements cationiques sont des produits déjà connus en eux-mêmes et sont par exemple décrits dans les brevets US 3 589 578 et US 4 031 307. De tels produits sont par ailleurs vendus notamment sous les dénominations commerciales de JAGUAR C13 S, JAGUAR C 15, JAGUAR C 17 et JAGUAR C162 par la société MEYHALL.

Selon une caractéristique préférée des compositions capillaires selon l'invention, ces dernières ne contiennent pas d'autres polymères cationiques que des gommes de guar cationiques.

### (ii)- Silicone(s):

Selon une caractéristique essentielle des compositions capillaires détergentes conformes à l'invention, ces dernières contiennent en outre au moins une silicone spécifique insoluble. De plus, cette silicone ne doit pas avoir été introduite dans la composition sous forme d'émulsion.

Selon la présente invention, par insoluble on entend insoluble dans la composition finale.

La viscosité de ces silicones insolubles est de préférence comprise entre 1000 et 350.000 cSt. et plus particulièrement entre 10.000 et 200.000 cSt ( entre 20 et 200 Pa.s) et encore plus préférentiellement entre 30.000 et 100.000 cSt.

La viscosité de ces silicones est mesurée à 25°C selon la norme ASTM 445 Appendice C.

Cette silicone est choisie parmi (i) les polydialkylsiloxanes, (ii) les polydiarylsiloxanes et (iii) les polyalkylarylsiloxanes.

Les radicaux alkyles comportent notamment de 1 à 10 atomes de carbone et désignent en particulier méthyle. Les radicaux aryles désignent plus particulièrement phényle.

Parmi les polydialkylsiloxanes, on peut citer principalement :
- les polydiméthylsiloxanes à groupements terminaux triméthylsilyle, comme par exemple, et à titre non limitatif, les huiles SILBIONE de la série 70047 commercialisées par RHONE POULENC, ou certaines VISCASIL de la GENERAL ELECTRIC (Viscosil 60.000), les FLUID DC 200 de la société DOW CORNING ou l'huile de silicone AK 300.000 de la société WACKER ;
- les polydiméthylsiloxanes à groupements terminaux hydroxydiméthylsilyle telles que les huiles de la série 48 V de RHONE POULENC ou le produit Q2-1401 commercialisé par la société DOW CORNING.

Dans cette classe de polydialkylsiloxanes, on peut également mentionner les polydialkylsiloxanes vendus par la société GOLDSCHMIDT sous les dénominations commerciales ABILWAX qui sont des polydiméthyldialkyl(C₁₀-C₂₀)siloxanes.

A titre indicatif, les compositions détergentes conformes à l'invention présentent généralement les compositions suivantes :
(i) tensioactif(s) anionique(s) : de 5 à 50 % en poids, de préférence de 5 à 20 % en poids, par rapport au poids total de la composition détergente ;
(ii) tensioactif(s) amphotère(s) de type alkylbétaïne : de 1 à 50 % en poids, de préférence de 1 à 20 % en poids, par rapport au poids total de la composition. De plus, la concentration en tensioactifs amphotères est généralement de 5 à 70 % en poids, et de préférence de 10 à 30 % en poids, par rapport au poids total du ou des tensioactifs anioniques présents dans la formulation détergente ;
(iii) gomme(s) de guar cationique(s) : de 0,001 % à 10 % en poids, de préférence de 0,005 % à 5 % en poids, et encore plus préférentiellement de 0,01 % à 3 % en poids, par rapport au poids total de la composition.
(iv) silicone(s) insoluble(s) non pré-émuisionnée(s) : de 0,05 % à 10 %, de préférence de 0,1 % à 5 % et encore plus préférentiellement de 0,2 % à 3%, par rapport au poids total de la composition.

Le véhicule, ou support, des compositions détergentes selon l'invention est de préférence de l'eau ou une solution hydroalcoolique d'un alcool inférieur tel que éthanol, isopropanol ou butanol.

Les compositions détergentes selon l'invention présentent un pH final généralement compris entre 3 et 9. De préférence, ce pH est compris entre 5 et 7. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions détergentes selon l'invention peuvent bien entendu contenir en outre tous les adjuvants usuels rencontrés dans le domaine des shampooings, comme par exemple des parfums, des agents conservateurs, des séquestrants, des épaississants, des adoucissants, des modificateurs de mousse, des colorants, des agents nacrants, des agents hydratants, des agents antipelliculaires ou anti-séborrhéiques, des vitamines, des filtres solaires, des agents de mise en suspension et autres.

Bien entendu, l'homme de l'art veillera à choisir ce ou ces éventuels composés complémentaires et/ou leurs quantités de manière telle que les propriétés avantageuses attachées intrinsèquement à l'association quaternaire (tensioactif anionique + tensioactif amphotère de type alkylbétaïne + gomme de guar cationique + silicone spécifique) conforme à l'invention ne soient pas, ou substantiellement pas, altérées par la ou les adjonctions envisagées.

Ces compositions peuvent se présenter sous la forme de liquides plus ou moins épaissis, de crèmes ou de gel et elles conviennent principalement au lavage, au soin et/ou le coiffage des cheveux.

Lorsque les compositions conformes à l'invention sont mises en oeuvre comme des shampooings classiques, elles sont simplement appliquées sur cheveux mouillés et la mousse générée par massage ou friction avec les mains est ensuite éliminée, après un éventuel temps de pause, par rinçage à l'eau, l'opération pouvant être répétée une ou plusieurs fois.

Comme indiqué précédemment, les compositions conformes à l'invention confèrent aux cheveux, après rinçage, un remarquable effet traitant qui se manifeste notamment par une facilité de démêlage, de coiffage, ainsi qu'un lissage et une douceur, nettement améliorés.

L'invention a également pour objet un procédé de lavage et de conditionnement des fibres kératiniques telles que les cheveux consistant à appliquer sur lesdites fibres mouillées une quantité efficace d'une composition telle que définie ci-dessus, puis à effectuer un rinçage à l'eau après un éventuel temps de pause.

Un exemple concret, mais nullement limitatif, illustrant l'invention va maintenant être donné.

### EXEMPLE

On a réalisé une composition de shampooing contenant :
- Lauryléthersulphate de sodium (C12/C14 à 70/30) à 2,2 moles d'oxyde d'éthylène (MA = matière active) 14 gMA
- Cocoyl bétaïne en solution aqueuse à 32% de matière active (DEHYTON AB 30 de HENKEL) 2,56 gMA
- Gomme de guar cationique (*) 0,05 g
- Silicone insoluble (**) 2,7 g
- Mélange (47/53 en poids) de 1-hexadécyloxy 2-octadécanol et d'alcool cétylique 2,5 g
- Monoisopropanolamide d'acides de coprah 1,6 g
- Acide citrique qs pH 5
- Eau déminéralisée qsp 100 g

(*) : gomme de guar modifiée par du chlorure de 2,3-époxypropyl triméthylammonium vendue sous la dénomination JAGUAR C13 S par la société RHONE POULENC
(**) : Polydiméthylsiloxane vendu sous la dénomination de VISCOSIL 60 000 Cst par la Société GENERAL ELECTRIC, utilisé et introduit tel quel dans la composition à préparer.

On effectue un shampooing en appliquant environ 12 g de la composition sur des cheveux préalablement mouillés. On fait mousser le shampooing puis on rince abondamment à l'eau.

Un panel d'experts a trouvé que les compositions conformes à l'invention confèrent aux cheveux, après rinçage, un remarquable effet traitant qui se manifeste notamment par une facilité de démêlage, de coiffage, ainsi qu'un lissage et une douceur remarquables des cheveux.

## Revendications

1. Procédé de préparation de compositions capillaires détergentes et conditionnantes, **caractérisées par** le fait les compositions comprennent, dans un milieu cosmétiquement acceptable,
(A) une base lavante comprenant au moins un tensioactif anionique et au moins un tensioactif amphotère choisis parmi les alkyl (C₁₀-C₁₄) bétaïnes de formule : 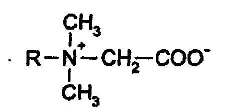 dans laquelle R désigne un radical alkyle, linéaire ou ramifié, en C₁₀-C₁₄, et,
(B) un système conditionneur comprenant au moins une gomme de guar cationique et au moins une silicone insoluble de viscosité inférieure ou égale à 350 Pa.s (350 000 cSt), choisie parmi (i) les polydialkylsiloxanes, (ii) les polydiarylsiloxanes et (iii) les polyalkylarylsiloxanes
et que ladite silicone est introduite dans la composition sous forme non émulsionnée.

2. Procédés selon la revendication 1, **caractérisées par le fait que** le ou lesdits tensioactifs anioniques sont présents à raison de 5 à 50 % en poids, de préférence de 5 à 20 % en poids, par rapport au poids total de la composition.

3. Procédés selon rune quelconque des revendications précédentes, **caractérisées par le fait que** le ou lesdits tensioactifs amphotères sont présents à raison de 1 à 50 % en poids, de préférence de 1 à 20 % en poids, par rapport au poids total de la composition.

4. Procédés selon l'une quelconque des revendications précédentes, **caractérisées par le fait que** le ou lesdits tensioactifs amphotères sont présents à raison de 5 à 70 % en poids, de préférence de 10 à 30 % en poids, par rapport au poids total du ou desdits tensioactifs anioniques.

5. Procédés selon l'une quelconque des revendications précédentes, **caractérisées par le fait que** ledit tensioactif amphotère est la cocoylbétaïne.

6. Procédés selon l'une quelconque des revendications précédentes,
**caractérisées par le fait que** ladite gomme de guar cationique est choisie parmi les gommes de guar modifiées par des groupements hydroxypropyl triméthylammonium.

7. Procédés selon l'une quelconque des revendications précédentes, **caractérisées par le fait que** lesdits polydialkylsiloxanes sont choisis dans le groupe constitué par :
- les polydiméthylsiloxanes à groupements terminaux triméthylsilyle,
- les polydiméthylsiloxanes à groupements terminaux hydroxydiméthyl silyle.

8. Procédés selon l'une quelconque des revendications précédentes, **caractérisées par le fait que** ladite gomme de guar cationique est présente à une teneur pondérale allant de 0,001 % à 10 % en poids par rapport au poids total de la composition.

9. Procédés selon la revendication 8, **caractérisées par le fait que** ladite teneur est comprise entre 0,005 % et 5 %.

10. Procédés selon la revendication 9, **caractérisées par le fait que** ladite teneur est comprise entre 0,01 % et 3 %.

11. Procédés selon l'une quelconque des revendications précédentes, **caractérisées par le fait que** ladite silicone insoluble est présente à une teneur pondérale comprise entre 0,05 % et 10 % en poids par rapport au poids total de la composition.

12. Procédés selon la revendication 11, **caractérisées par le fait que** ladite teneur est comprise entre 0,1 % et 5 %.

13. Procédés selon la revendication 12, **caractérisées par le fait que** ladite teneur est comprise entre 0,2 % et 3%.

14. Procédés selon rune quelconque- des revendications précédentes, **caractérisées par le fait qu'**elles présentent un pH compris entre 3 et 9.

15. Procédés selon l'une quelconque des revendications précédentes, **caractérisées par le fait qu'**il s'agit de compositions aqueuses ou hydroalcooliques.

16. Procédés selon l'une quelconque des revendications précédentes, **caractérisées en ce que** ladite base lavante est exempte de tensioactifs autres que des tensioactifs anioniques et des tensioactifs amphotères de type alkylbétaïne en C₁₀-C₁₄.

17. Procédés selon l'une quelconque des revendications précédentes, **caractérisées en ce que** ledit système conditionneur est exempt de polymères cationiques autres que des gommes de guar cationiques.

18. Procédé de lavage et de conditionnement des fibres kératiniques telles que les cheveux consistant à appliquer sur lesdites fibres mouillées une quantité efficace d'une composition obtenue selon le procédé défini à l'une quelconque des revendications précédentes, puis à effectuer un rinçage à l'eau après un éventuel temps de pause.

19. Utilisation d'une composition obtenue selon le procédé défini à l'une quelconque des revendications 1 à 17 pour le nettoyage et/ou le conditionnement des cheveux.

## Patentansprüche

1. Verfahren zur Herstellung von reinigenden und konditionierenden Zusammensetzungen für das Haar, **dadurch gekennzeichnet, dass** die Zusammensetzungen in einem kosmetisch akzeptablen Medium enthalten:
(A) eine reinigende Basisformulierung, die mindestens einen anionischen grenzflächenaktiven Stoff und mindestens einen amphoteren grenzflächenaktiven Stoff enthält, der unter den C₁₀₋₁₄-Alkylbetainen der folgenden Formel ausgewählt ist: 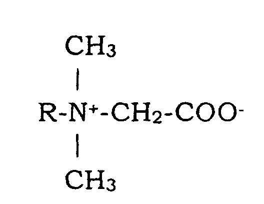 worin R eine geradkettige oder verzweigte C₁₀₋₁₄-Alkylgruppe bedeutet, und
(B) ein Konditioniersystem, das mindestens ein kationisches Guargummi und mindestens ein unlösliches Silicon mit einer Viskosität von 350 Pa·s oder darunter (höchstens 350 000 cSt) enthält, das unter (i) den Polydialkylsiloxanen, (ii) den Polydiarylsiloxanen und (iii) den Polyalkylarylsiloxanen ausgewählt ist, wobei das Silicon in nicht emulgierter Form in die Zusammensetzung gegeben wird.

2. Verfahren nach Anspruch 1, **dadurch gekennzeichnet, dass** der oder die anionischen grenzflächenaktiven Stoffe in Mengenanteilen von 5 bis 50 Gew.-% und vorzugsweise 5 bis 20 Gew.-%, bezogen auf das Gesamtgewicht der Zusammensetzung, vorliegen.

3. Verfahren nach einem der vorhergehenden Ansprüche, **dadurch gekennzeichnet, dass** der oder die amphoteren grenzflächenaktiven Stoffe in Mengenanteilen von 1 bis 50 Gew.-% und vorzugsweise 1 bis 20 Gew.-%, bezogen auf das Gesamtgewicht der Zusammensetzung, vorliegen.

4. Verfahren nach einem der vorhergehenden Ansprüche, **dadurch gekennzeichnet, dass** der oder die amphoteren grenzflächenaktiven Stoffe in Mengenanteilen von 5 bis 70 Gew.-% und vorzugsweise 10 bis 30 Gew.-%, bezogen auf das Gesamtgewicht des oder der anionischen grenzflächenaktiven Stoffe, vorliegen.

5. Verfahren nach einem der vorhergehenden Ansprüche, **dadurch gekennzeichnet, dass** der amphotere grenzflächenaktive Stoff das Cocoylbetain ist.

6. Verfahren nach einem der vorhergehenden Ansprüche, **dadurch gekennzeichnet, dass** das kationische Guargummi unter den kationischen Guargummen ausgewählt ist, die mit Hydroxypropyltrimethylammonium-Gruppen modifiziert wurden.

7. Verfahren nach einem der vorhergehenden Ansprüche, **dadurch gekennzeichnet, dass** die Polydialkylsiloxane ausgewählt sind unter:
- Polydimethylsiloxanen mit Trimethylsilylendgruppen, und
- Polydimethylsiloxanen mit Hydroxydimethylsilylendgruppen.

8. Verfahren nach einem der vorhergehenden Ansprüche, **dadurch gekennzeichnet, dass** das kationische Guargummi in Mengenanteilen von 0,001 bis 10 Gew.-%, bezogen auf das Gesamtgewicht der Zusammensetzung, vorliegt.

9. Verfahren nach Anspruch 8, **dadurch gekennzeichnet, dass** der Mengenanteil im Bereich von 0,005 bis 5 % liegt.

10. Verfahren nach Anspruch 9, **dadurch gekennzeichnet, dass** der Mengenanteil im Bereich von 0,01 bis 3 % liegt.

11. Verfahren nach einem der vorhergehenden Ansprüche, **dadurch gekennzeichnet, dass** das unlösliche Silicon in einer Gewichtsmenge von 0,05 bis 10 Gew.-%, bezogen auf das Gesamtgewicht der Zusammensetzung, vorliegt.

12. Verfahren nach Anspruch 11, **dadurch gekennzeichnet, dass** der Mengenanteil im Bereich von 0,1 bis 5 % liegt.

13. Verfahren nach Anspruch 12, **dadurch gekennzeichnet, dass** der Mengenanteil im Bereich von 0,2 bis 3 % liegt.

14. Verfahren nach einem der vorhergehenden Ansprüche, **dadurch gekennzeichnet, dass** die Zusammensetzungen einen pH-Wert im Bereich von 3 bis 9 aufweisen.

15. Verfahren nach einem der vorhergehenden Ansprüche, **dadurch gekennzeichnet, dass** es sich um wässrige oder wässrig-alkoholische Zusammensetzungen handelt.

16. Verfahren nach einem der vorhergehenden Ansprüche, **dadurch gekennzeichnet, dass** die reinigende Basisformulierung nur anionische grenzflächenaktive Stoffe und amphotere grenzflächenaktive Stoffe vom Typ C₁₀₋₁₄-Alkylbetain und keine anderen grenzflächenaktive Stoffe enthält.

17. Verfahren nach einem der vorhergehenden Ansprüche, **dadurch gekennzeichnet, dass** das Konditioniersystem nur kationische Guargummen und keine anderen kationischen Polymere enthält.

18. Verfahren zum Waschen und zum Konditionieren von Keratinfasern, wie dem Haar, das darin besteht, auf die feuchten Fasern eine wirksame Menge einer Zusammensetzung aufzutragen, die gemäß dem Verfahren nach einem der vorhergehenden Ansprüche hergestellt wurde, und anschließend, gegebenenfalls nach einer Einwirkzeit, mit Wasser zu spülen.

19. Verwendung einer Zusammensetzung, die gemäß dem Verfahren nach einem der Ansprüche 1 bis 17 hergestellt wurde, zur Reinigung und/oder zum Konditionieren der Haare.

## Claims

1. Process for preparing detergent and conditioning hair-care compositions, **characterized in that** the compositions comprise, in a cosmetically acceptable medium,
(A) a washing base comprising at least one anionic surfactant and at least one amphoteric surfactant chosen from (C₁₀-C₁₄) alkylbetaines of formula: 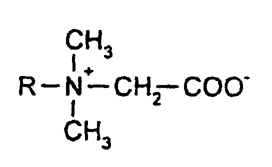 in which R denotes a linear or branched C₁₀-C₁₄ alkyl radical, and
(B) a conditioning system comprising at least one cationic guar gum and at least one insoluble silicone of viscosity less than or equal to 350 Pa.s (350,000 cSt), chosen from (i) polydialkylsiloxanes, (ii) polydiarylsiloxanes and (iii) polyalkylarylsiloxanes,
and **in that** the said silicone is introduced into the composition in non-emulsified form.

2. Processes according to Claim 1, **characterized in that** the said anionic surfactant or surfactants is/are present in the proportion of from 5 to 50% by weight, and preferably from 5 to 20% by weight, relative to the total weight of the composition.

3. Processes according to either of the preceding claims, **characterized in that** the said amphoteric surfactant or surfactants is/are present in the proportion of from 1 to 50% by weight, and preferably from 1 to 20% by weight, relative to the total weight of the composition.

4. Processes according to any one of the preceding claims, **characterized in that** the said amphoteric surfactant or surfactants is/are present in the proportion of from 5 to 70% by weight, and preferably from 10 to 30% by weight, relative to the total weight of the said anionic surfactant or surfactants.

5. Processes according to any one of the preceding claims, **characterized in that** the said amphoteric surfactant is cocoylbetaine.

6. Processes according to any one of the preceding claims, **characterized in that** the said cationic guar gum is chosen from guar gums modified with hydroxypropyltrimethylammonium groups.

7. Processes according to any one of the preceding claims, **characterized in that** the said polydialkylsiloxanes are chosen from the group consisting of:
- polydimethylsiloxanes containing terminal trimethylsilyl groups,
- polydimethylsiloxanes containing terminal hydroxydimethylsilyl groups.

8. Processes according to any one of the preceding claims, **characterized in that** the said cationic guar gum is present in a weight content ranging from 0.001% to 10% by weight relative to the total weight of the composition.

9. Processes according to Claim 8, **characterized in that** the said content is between 0.005% and 5%.

10. Processes according to Claim 9, **characterized in that** the said content is between 0.01% and 3%.

11. Processes according to any one of the preceding claims, **characterized in that** the said insoluble silicone is present in a weight content of between 0.05% and 10% by weight relative to the total weight of the composition.

12. Processes according to Claim 11, **characterized in that** the said content is between 0.1% and 5%.

13. Processes according to Claim 12, **characterized in that** the said content is between 0.2% and 3%.

14. Processes according to. any one of the preceding claims, **characterized in that** they have a pH of between 3 and 9.

15. Processes according to any one of the preceding claims, **characterized in that** they are aqueous or aqueous-alcoholic compositions.

16. Processes according to any one of the preceding claims, **characterized in that** the said washing base is free from surfactants other than anionic surfactants and amphoteric surfactants of the C₁₀-C₁₄ alkylbetaine type.

17. Processes according to any one of the preceding claims, **characterized in that** the said conditioning system is free from cationic polymers other than cationic guar gums.

18. Process for washing and conditioning keratinous fibres such as the hair, consisting in applying to the said fibres in the wet state an effective amount of a composition obtained according to the process defined in any one of the preceding claims, and in then rinsing with water after an optional period of exposure.

19. Use of a composition obtained according to the process defined in any one of Claims 1 to 17 for cleansing and/or conditioning the hair.
